# NOUVEAU FASCICULE DE BREVET EUROPEEN

(11) **EP 1 291 001 B2**
(45) Date de publication et mention de la décision concernant l'opposition: **24.01.2018**
(45) Mention de la délivrance du brevet: 21.02.2007
(21) Numéro de dépôt: 02292000.3
(22) Date de dépôt: 08.08.2002
(51) Int. Cl.: A61K 8/81, A61Q 5/00

(54) **Compositions cosmétiques contenant un copolymère d'acide méthacrylique, une huile et leurs utilisations**
Ein Copolymer aus Methacrylsäure und ein Öl enthaltende kosmetische Zusammensetzungen und deren Verwendungen
Cosmetic compositions containing a copolymer of methacrylic acid and an oil and uses thereof

(30) Priorité: 11.09.2001 FR 0111743
(43) Date de publication de la demande: 12.03.2003
(73) Titulaire: L'Oréal, 75008 Paris (FR)
(72) Inventeur: Maubru, Mireille, 78400 Chatou (FR)
(74) Mandataire: Martin-Charbonneau, Virginie

(56) Documents cités:
- EP-A- 0 761 095
- FR-A1- 2 710 263
- GB-A- 1 110 240
- US-A- 6 132 705
- DATABASE CHEMICAL ABSTRACTS [en ligne] retrieved from STN Database accession no. 123: 152585 XP002206903 & JP 07 145023 A (LION CORP.) 6 juin 1995 (1995-06-06)
- Carbopol Aqua SF-1 Polymer, Body Lotion Formulation, December 2000
- Carbopol Aqua SF-1 Polymer, Clear Facial Cleaner Formulation.
- Carbopol Aqua SF-1 Polymer, Facial Cream Formulation, December 2000
- Carbopol Aqua SF-1 Polymer, Alpha Hydroxy Acid Cream Formulation, December 2000
- Carbopol Aqua SF-1 Polymer, Formulation Summary Sheet, January 2001
- Carbopol Aqua SF-1 Polymer, Product Summary Sheet, December 2000
- Carbopol AQUA SF-1 Polymer Pearlized 3-in-1 Conditioning Shampoo Formulation December 2000

## Description

La présente invention concerne de nouvelles compositions cosmétiques comprenant dans un milieu cosmétiquement acceptable au moins un copolymère réticulé acide méthacrylique / acrylate d'alkyle en C₁-C₄, au moins une huile choisie parmi les huiles synthétiques choisies parmi les polyoléfines, les esters d'acides en C₃-C₁₄ et comprenant au total moins de 20 atomes de carbone, les huiles végétales ayant une teneur en acide palmitoléique supérieure ou égal à 0,2% en poids de l'huile, et un polymère cationique.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

On a déjà préconisé dans les compositions pour le lavage ou le soin des matières kératiniques telles que les cheveux l'utilisation d'agents conditionneurs, notamment des polymères cationiques ou des silicones, pour faciliter le démêlage des cheveux et pour leur communiquer douceur et souplesse. Cependant, les avantages cosmétiques mentionnés ci-avant s'accompagnent malheureusement également, sur cheveux séchés, de certains effets cosmétiques jugés indésirables, à savoir un alourdissement de la coiffure (manque de légéreté du cheveu), un manque de lissage (cheveu non homogène de la racine à la pointe).

En outre, l'usage des polymères cationiques dans ce but présente divers inconvénients. En raison de leur forte affinité pour les cheveux, certains de ces polymères se déposent de façon importante lors d'utilisations répétées, et conduisent à des effets indésirables tel qu'un toucher désagréable, chargé, un raidissement des cheveux, et une adhésion interfibres affectant le coiffage. Ces inconvénients sont accentués dans le cas de cheveux fins, qui manquent de nervosité et de volume.

On a déjà proposé d'utiliser en tant qu'agent conditionneur des huiles telles que les huiles végétales ou animales ou encore des esters d'acides gras. Cependant, les matières kératiniques traitées avec ces compositions présentent le plus souvent un toucher gras rédhibitoire.

De plus, pour épaissir et stabiliser les compositions contenant des agents conditionneurs insolubles, des agents de stabilisation tels que les polymères acryliques réticulés du type Carbopol sont fréquemment utilisés. Néanmoins, ces agents de stabilisation présentent l'inconvénient de diminuer les performances cosmétiques des shampooings, notamment en rendant les cheveux plus chargés et plus rêches.

En résumé, il s'avère que les compositions cosmétiques actuelles contenant des huiles, ne donnent pas encore complètement satisfaction.

On connaît dans l'état de la technique des compositions cosmétiques en particulier détergentes contenant un copolymère d'acide méthacrylique et d'acrylate d'alkyle comme agent de stabilisation ou de suspension d'ingrédients insolubles dans l'eau comme des silicones ou des corps gras. De telles compositions ont été décrites notamment dans la demande de brevet WO01/76552. Les qualités de mousse et les propriétés cosmétiques obtenues avec ces compositions ne sont pas encore suffisamment satisfaisantes.

La demanderesse a maintenant découvert que l'association d'un copolymère réticulé acide méthacrylique / acrylate d'alkyle en C₁-C₄, d'une huile particulière et d'un polymère cationique permet de remédier à ces inconvénients.

En effet, il a été constaté que l'utilisation dudit copolymère acrylique dans les compositions de la présente invention permettait d'obtenir sur les matières kératiniques, notamment les cheveux de très bonnes propriétés cosmétiques particulièrement une facilité de démêlage, ainsi qu'un apport de légèreté, de lissage, de douceur et de souplesse sans aucune sensation de toucher chargé.

Par ailleurs, les compositions selon l'invention sont stables et présentent un aspect visuel esthétique. Les propriétés d'usage (aspect, consistance, abondance de la mousse, élimination de la mousse) sont très satisfaisantes.

Par ailleurs, les compositions de l'invention appliquées sur la peau notamment sous forme de bain moussant ou de gel douche, apportent une amélioration de la douceur de la peau.

Ainsi, selon la présente invention, il est maintenant proposé de nouvelles compositions cosmétiques, comprenant, dans un milieu cosmétiquement acceptable, au moins un copolymère réticulé d'acide méthacrylique et d'acrylate d'alkyle en C₁-C₄, au moins une huile choisie parmi les huiles synthétiques choisies parmi les polyoléfines, les esters d'acides en C₃-C₁₄ et comprenant au total moins de 20 atomes de carbone, les huiles végétales ayant une teneur en acide palmitoléique supérieure ou égal à 0,2% en poids de l'huile, et un polymère cationique.

L'invention a également pour objet l'utilisation d'une composition telle que définie ci-dessus pour apporter de la légèreté, de la douceur, du lissage au toucher, de la souplesse aux cheveux.

Un autre objet de l'invention concerne un procédé de traitement des matières kératiniques, telles que les cheveux, caractérisé en ce qu'il consiste à appliquer sur lesdites matières des compositions cosmétiques selon l'invention.

Selon la présente invention, par matières kératiniques, on comprend les cheveux, les cils, les sourcils, la peau, les ongles, les muqueuses ou le cuir chevelu et plus particulièrement les cheveux.

Un autre objet de l'invention concerne l'utilisation d'un copolymère réticulé acide méthacrylique / acrylate d'alkyle en C₁-C₄ dans, ou pour la fabrication d'une composition cosmétique comprenant une huile telle que définie ci-dessous et un polymère cationique.

Les différents objets de l'invention vont maintenant être détaillés. L'ensemble des significations et définitions des composés utilisés dans la présente invention données ci-dessous sont valables pour l'ensemble des objets de l'invention.

L'une des caractéristiques essentielles de l'invention est la présence d'un copolymère réticulé d'acide méthacrylique et d'acrylate d'alkyle en C₁-C₄.

L'acide méthacrylique est présent de préférence dans des quantités allant de 20 à 80% en poids et plus particulièrement de 25 à 70 % en poids et encore plus particulièrement de 35 à 60% en poids par rapport au poids total du copolymère.

L'acrylate d'alkyle est présent de préférence dans des quantités allant de 15 à 80% en poids et plus particulièrement de 25 à 75 % en poids et encore plus particulièrement de 40 à 65% en poids par rapport au poids total du copolymère. Il est choisi notamment parmi l'acrylate de méthyle, l'acrylate d'éthyle ou l'acrylate de butyle et plus particulièrement l'acrylate d'éthyle.

Ce copolymère est partiellement ou totalement réticulé par au moins un agent réticulant classique. Les agents réticulants sont notamment des composés polyinsaturés, en particulier éthylèniquement polyinsaturés. Ces composés sont notamment les polyalcényléthers de sucrose ou de polyols, les diallylphtalates, le divinylbenzene, le (méth)acrylate d'allyle, di(méth)acrylate d'éthylèneglycol, le méthylène bis-acrylamide, le tri(méth)acrylate de triméthylol propane, l'itaconate de diallyle, le fumarate de diallyle, le maléate de diallyle, le (méth)acrylate de zinc, les dérivés d'huile de ricin ou de polyols fabriqués à partir d'acides carboxyliques insaturés.

Comme agent réticulant, on peut également utiliser des composés monomères insaturés et comportant un groupe réactif susceptible de réagir avec une insaturation pour former un copolymère réticulé.

La teneur en agent réticulant varie en général de 0,01 à 5% en poids et de préférence de 0,03 à 3% en poids et encore plus particulièrement de 0,05 à 1% en poids par rapport au poids total du copolymère.

Selon une forme particulièrement préférée, le copolymère de l'invention peut se présenter notamment sous forme de dispersion dans l'eau. La taille moyenne en nombre des particules de copolymère dans la dispersion est généralement comprise entre 10 et 500 nm et de préférence entre 20 et 200 nm et plus préférentiellement de 50 à 150 nm.

Ces copolymères sont notamment décrit dans la demande WO01/76552.

On utilisera plus particulièrement le copolymère acide méthacrylique/acrylate d'éthyle réticulé sous forme de dispersion aqueuse à 30% fabriqué et vendu sous le nom CARBOPOL AQUA SF-1 par la société NOVEON.

La concentration en copolymère est généralement comprise entre 0,01 et 20% en poids par rapport au poids total de la composition et de préférence entre 0,05 et 15% en poids.

Les huiles de synthèse sont choisies parmi les polyoléfines, en particulier les poly-α-oléfines et plus particulièrement :
- de type polybutène, hydrogéné ou non, et de préférence polyisobutène, hydrogéné ou non.

On utilise de préférence les oligomères d'isobutylène de poids molaire inférieur à 1000 et leurs mélange avec des polyisobutylènes de poids molaire supérieur à 1000 et de préférence compris entre 1000 et 15000.

A titre d'exemples de poly-α-oléfines utilisables dans le cadre de la présente invention, on peut plus particulièrement mentionner les polyisobutènes vendus sous le nom de PERMETHYL 99 A, 101 A, 102 A , 104 A (n=16) et 106 A (n=38) par la Société PRESPERSE Inc, ou bien encore les produits vendus sous le nom de ARLAMOL HD (n=3) par la Société ICI (n désignant le degré de polymérisation),
- de type polydécène, hydrogéné ou non.

De tels produits sont vendus par exemple sous les dénominations ETHYLFLO par la société ETHYL CORP., et d'ARLAMOL PAO par la société ICI.

Les huiles végétales sont choisies préférentiellement dans le groupe formé par les huiles d'avocat, de jojoba, d'olive, d'abricot et, les huiles végétales transestérifiées.

Les esters utilisables selon l'invention sont notamment choisis parmi les esters d'acides mono- di-, tri-carboxyliques aliphatiques saturés ou insaturés, linéaires ou ramifiés en C₃-C₁₄ et de monoalcools ou de polyols aliphatiques, saturés ou insaturés, linéaires ou ramifiés en C₁-C₁₆, le nombre total de carbone étant inférieur ou égal à 20.

Parmi les esters selon l'invention, on peut citer le lactate de cétyle ; le lactate d'alkyle en C₁₂-C₁₅; le lactate de lauryle ; le lactate d'oléyle ; les myristates d'alkyles en C₁-C₅ tels que le myristate d'isopropyle, de butyle, le laurate d'hexyle, l'isononate d'isononyle, le néopentanoate d'isodécyle.

La ou les huiles sont utilisées de préférence en une quantité comprise entre 0,001 et 20% en poids par rapport au poids total de la composition. Plus préférentiellement, cette quantité est comprise entre 0,05 et 15% en poids par rapport au poids total de la composition et encore plus préférentiellement entre 0,01 et 10%, voire 0,1 et 10% en poids.

La composition comprend en outre un polymère cationique.

Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A-0 337 354 et dans les demandes de brevets français FR-A-2 270 846, 2 383 660, 2 598 611, 2 470 596 et 2 519 863.

De manière encore plus générale, au sens de la présente invention, l'expression "polymère cationique" désigne tout polymère contenant des groupements cationiques et/ou des groupements ionisables en groupements cationiques.

Les polymères cationiques préférés sont choisis parmi ceux qui contiennent des motifs comportant des groupements amines primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse molaire moyenne en nombre ou en poids comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :
(1) les homopolymères ou copolymères dérivés d'esters ou d'amides acryliques ou méthacryliques et comportant au moins un des motifs de formules suivantes: 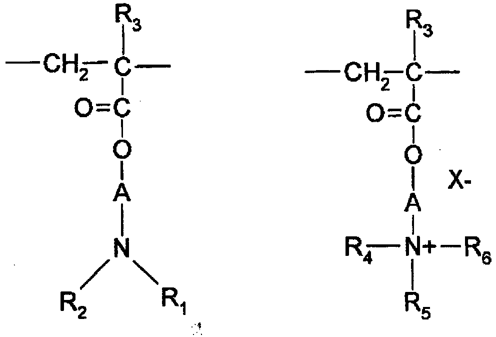 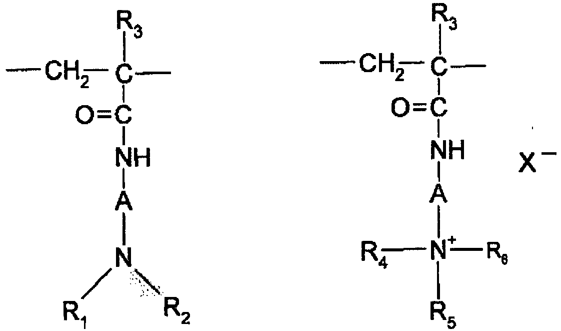 dans lesquelles:
   R₃, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   X désigne un anion dérivé d'un acide minéral ou organique tel que un anion méthosulfate ou un halogénure tel que chlorure ou bromure.

   Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.
   Ainsi, parmi ces copolymères de la famille (1), on peut citer:
   - les copolymères d'acrylamide et de diméthylaminoéthyl méthacrylate quaternisé au sulfate de diméthyle ou avec un halogénure de diméthyle tels que celui vendu sous la dénomination HERCOFLOC par la société HERCULES,
   - les copolymères d'acrylamide et de chlorure de méthacryloyloxyéthyltriméthyl-ammonium décrit par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthyl-ammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactame/ vinylpyrrolidone tel que le produit vendu sous la dénomination GAFFIX VC 713 par la société ISP,
   - les copolymères vinylpyrrolidone / méthacrylamidopropyl dimethylamine commercialisés notamment sous la dénomination STYLEZE CC 10 par ISP.
   - et les copolymères vinylpyrrolidone / méthacrylamide de diméthylaminopropyle quaternisé tel que le produit vendu sous la dénomination "GAFQUAT HS 100" par la société ISP.
(2) Les polysaccharides cationiques notamment les celluloses et les gommes de galactomannanes cationiques. Parmi les polysaccharides cationiques, on peut citer plus particulièrement les dérivés d'éthers de cellulose comportant des groupements ammonium quaternaires, les copolymères de cellulose cationiques ou les dérivés de cellulose greffés avec un monomère hydrosoluble d'ammonium quaternaire et les gommes de galactomannanes cationiques.
   Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société NALCO. Ces polymères sont également définis dans le dictionnaire CTFA comme des ammonium quaternaires d'hydroxyéthylcellulose ayant réagi avec un époxyde substitué par un groupement triméthylammonium.
   Les copolymères de cellulose cationiques ou les dérivés de cellulose greffés avec un monomère hydrosoluble d'ammonium quaternaire, sont décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
   Les produits commercialisés répondant à cette définition sont plus particulièrement les produits vendus sous la dénomination "Celquat L 200" et "Celquat H 100" par la Société National Starch.
   Les gommes de galactomannane cationiques sont décrites plus particulièrement dans les brevets US 3 589 578 et 4 031 307 en particulier les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société MEYHALL.
(3) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;
(4) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;
(5) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(6) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(7) les cyclopolymères d'alkyl diallyl amine ou de dialkyl diallyl ammonium tels que les homopolymères ou copolymères comportant comme constituant principal de la chaîne des motifs répondant aux formules (I) ou (I') : 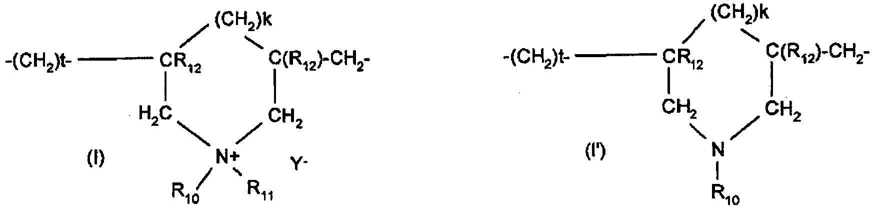
   formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C1-C4) ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   R₁₀ et R₁₁, indépendamment l'un de l'autre, désignent de préférence un groupement alkyle ayant de 1 à 4 atomes de carbone.

   Parmi les polymères définis ci-dessus, on peut citer plus particulièrement l'homopolymère de chlorure de diméthyldiallylammonium vendu sous la dénomination "MERQUAT 100" par la société NALCO (et ses homologues de faibles masses molaires moyenne en poids) et les copolymères de chlorure de diallyldiméthylammonium et d'acrylamide .
(8) les polymères de diammonium quaternaire contenant des motifs récurrents répondant à la formule : 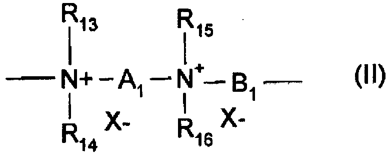 formule (II) dans laquelle :
   R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote ou bien R₁₃, R₁₄, R₁₅ et R₁₆ représentent un radical alkyle en C1-C6 linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
   dans lequel D désigne :
   a) un reste de glycol de formule : -O-Z-O-, où Z désigne un radical hydrocarboné linéaire ou ramifié ou un groupement répondant à l'une des formules suivantes :

      -(CH2-CH2-O)x-CH2-CH2-

      -[CH2-CH(CH3)-O]y-CH2-CH(CH3)-

      où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule : -NH-Y-NH-, où Y désigne un radical hydrocarboné linéaire ou ramifié, ou bien le radical bivalent

      -CH2-CH2-S-S-CH2-CH2- ;
   d) un groupement uréylène de formule : -NH-CO-NH-;

   De préférence, X⁻ est un anion tel que le chlorure ou le bromure.
   Ces polymères ont une masse molaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule : 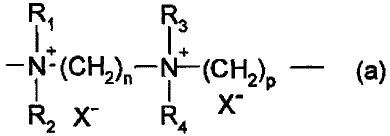 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.
   Un composé de formule (a) particulièrement préféré est celui pour lequel R₁, R₂, R₃ et R₄, représentent un radical méthyle et n = 3, p = 6 et X = Cl, dénommé Hexadimethrine chloride selon la nomenclature INCl (CTFA).
(9) les polymères de polyammonium quaternaires comprenant des motifs de formule (III): 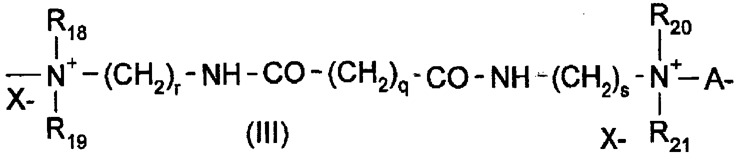 formule dans laquelle :
   R₁₈, R₁₉, R₂₀ et R₂₁, identiques ou différents, représentent un atome d'hydrogène ou un radical méthyle, éthyle, propyle, β-hydroxyéthyle, β-hydroxypropyle ou -CH₂CH₂(OCH₂CH₂)ₚOH,
   où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X⁻ désigne un anion tel qu'un halogènure,
   A désigne un radical d'un dihalogénure ou représente de préférence -CH₂-CH₂-O-CH₂-CH₂-.

   De tels composés sont notamment décrits dans la demande de brevet EP-A-122 324.
   On peut par exemple citer parmi ceux-ci, les produits "Mirapol® A 15", "Mirapol® AD1", "Mirapol® AZ1" et "Mirapol® 175" vendus par la société Miranol.
(10) Les polymères quaternaires de vinylpyrrolidone et de vinylimidazole tels que par exemple les produits commercialisés sous les dénominations Luviquat® FC 905, FC 550 et FC 370 par la société B.A.S.F.
(11) Les polyamines comme le Polyquart® H vendu par COGNIS, référencé sous le nom de « POLYETHYLENEGLYCOL (15) TALLOW POLYAMINE » dans le dictionnaire CTFA.
(12) Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE® SC 92 » par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de « SALCARE® SC 95 » et « SALCARE® SC 96 » par la Société ALLIED COLLOIDS.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les dérivés d'éther de cellulose quaternaires tels que les produits vendus sous la dénomination « JR 400 » par la Société NALCO, les cyclopolymères cationiques, en particulier les homopolymères ou copolymères de chlorure de diméthyldiallylammonium, vendus sous les dénominations « MERQUAT 100 », « MERQUAT 550 » et « MERQUAT S » par la société NALCO, les polymères quaternaires de vinylpyrrolidone et de vinylimidazole, les homopolymères ou copolymères réticulés de sels de méthacryloyloxyalkyl(C₁-C₄) trialkyl(C₁-C₄)ammonium et leurs mélanges.

Selon l'invention, le ou les polymères cationiques peuvent représenter de 0,001 % à 20 % en poids, de préférence de 0,01 % à 10% en poids et plus particulièrement de 0,1 à 5% en poids par rapport au poids total de la composition finale.

Les compositions de l'invention contiennent en outre avantageusement au moins un agent tensioactif qui est généralement présent en une quantité comprise entre 0,01% et 50% en poids environ, de préférence entre 0,1% et 40% et encore plus préférentiellement entre 0,5% et 30%, par rapport au poids total de la composition.

Cet agent tensioactif peut être choisi parmi les agents tensioactifs anioniques, amphotères, non-ioniques, cationiques ou leurs mélanges.

Les tensioactifs convenant à la mise en oeuvre de la présente invention sont notamment les suivants :
(i) Tensioactif(s) anionique(s) :
   Leur nature ne revêt pas, dans le cadre de la présente invention, de caractère véritablement critique.

   Ainsi, à titre d'exemple de tensioactifs anioniques utilisables, seuls ou mélanges, dans le cadre de la présente invention, on peut citer notamment (liste non limitative) les sels (en particulier sels de métaux alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryle désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₈-C₂₄) éther carboxyliques polyoxyalkylénés, les acides alkyl(C₈-C₂₄)aryl éther carboxyliques polyoxyalkylénés,les acides alkyl(C₆-C₂₄) amido éther carboxyliques polyoxyalkylénés et leurs sels, en particulier ceux comportant de 2 à 50 groupements oxyde d'éthylène, et leurs mélanges.
   Parmi les tensioactifs anioniques, on préfère utiliser selon l'invention les sels d'alkylsulfates et d'alkyléthersufates et leurs mélanges.
(ii) Tensioactif(s) non ionique(s) :
   Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensioactifs non-ioniques rentrant particulièrement bien dans le cadre de la présente invention.
(iii) Tensioactif(s) amphotère(s):
   Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁₋C₆) bétaïnes ou les alkyl (C₈-C₂₀) amidoalkyl (Ci-Ce) sulfobétaïnes.

   Parmi les alkyl (C₈-C₂₀) amidoalkyl (C₁-C₆) bétaïnes on peut citer la cocoamidopropylbétaïne vendue notamment par GOLDSCHMIDT sous le nom de TEGOBETAINE F50.
   Parmi les dérivés d'amines, on peut citer les produits commercialisés sous les dénomination MIRANOL, tels que décrits dans les brevets US-2 528 378 et US-2 781 354 et de structures :

   R₂-CONHCH₂CH₂-N(R₃)(R₄)(CH₂COO-) (2)

   dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
   et

   R₅-CONHCH₂CH₂-N(B)(C) (3)

   dans laquelle :
   B représente -CH₂CH₂OX', C représente -(CH₂)_{z} -Y', avec z = 1 ou 2,
   X' désigne le groupement -CH₂CH₂₋COOH ou un atome d'hydrogène
   Y' désigne -COOH ou le radical -CH₂ - CHOH - SO3H
   R₅ désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

   Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Coco-amphodipropionate, Disodium Lauroamphodipropionate, Disodium Caprylampho-dipropionate, Disodium Capryloamphodipropionate, Lauroamphodipropionic acid, Coco-amphodipropionic acid.
   A titre d'exemple on peut citer le cocoamphodiacetate commercialisé sous la dénomination commerciale MIRANOL C2M concentré par la société RHODIA CHIMIE.
(iv) Les tensioactifs cationiques peuvent être choisis parmi :
   A) les sels d'ammonium quaternaires de la formule générale (XIII) suivante : 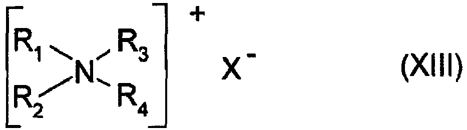 dans laquelle X est un anion choisi dans le groupe des halogénures (chlorure, bromure ou iodure) ou alkyl(C₂-C₈)sulfates plus particulièrement méthylsulfate, des phosphates, des alkyl-ou-alkylarylsulfonates, des anions dérivés d'acide organique tel que l'acétate ou le lactate.
      , et
      a) les radicaux R1 à R3, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide,
         R4 désigne un radical alkyle, linéaire ou ramifié, comportant de 16 à 30 atomes de carbone.
         De préférence le tensioactif cationique est un sel (par exemple chlorure) de béhényl triméthyl ammonium.
      b) les radicaux R1 et R2, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide et hydroxyalkyle, comportant environ de 1 à 4 atomes de carbone;
         R3 et R4, identiques ou différents, désignent un radical alkyle, linéaire ou ramifié, comportant de 12 à 30 atomes de carbone, ledit radical comprenant au moins une fonction ester ou amide.
         R3 et R4 sont notamment choisis parmi les radicaux alkyl(C₁₂-C₂₂)amido alkyle(C₂-C₆), alkyl(C₁₂-C₂₂)acétate ;
         De préférence le tensioactif cationique est un sel (par exemple chlorure) de stéaramidopropyl diméthyl (myristylacétate) ammonium.
   B) - les sels d'ammonium quaternaire de l'imidazolinium, comme par exemple celui de formule (XIV) suivante : 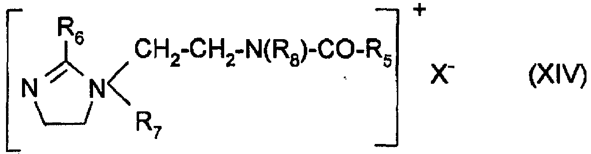 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄, R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple le Quaternium-27(CTFA 1997) ou le Quaternium-83 (CTFA 1997) commercialisés sous les dénominations "REWOQUAT" W 75, W90, W75PG, W75HPG par la société WITCO,
   C) - les sels de diammonium quaternaire de formule (XV) : 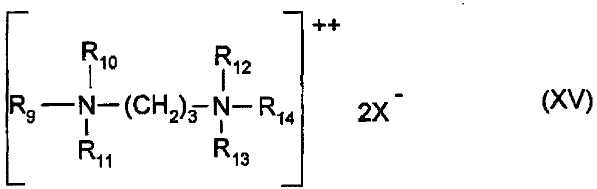 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
   D) - les sels d'ammonium quaternaire contenant au moins une fonction ester de formule (XVI) suivante : 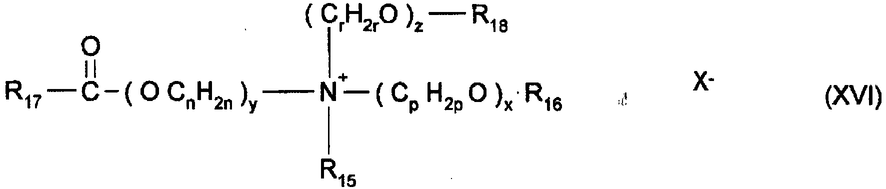 dans laquelle :
      - R₁₅ est choisi parmi les radicaux alkyles en C₁-C₆ et les radicaux hydroxyalkyles ou dihydroxyalkyles en C₁-C₆ ;
      - R₁₆ est choisi parmi :
         - le radical 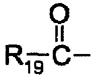
         - les radicaux R₂₀ hydrocarbonés en C₁-C₂₂ linéaires ou ramifiés, saturés ou insaturés,
         - l'atome d'hydrogène,
      - R₁₈ est choisi parmi :
         - le radical 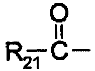
         - les radicaux R₂₂ hydrocarbonés en C₁-C₆ linéaires ou ramifiés, saturés ou insaturés,
         - l'atome d'hydrogène,
      - R₁₇, R₁₉ et R₂₁, identiques ou différents, sont choisis parmi les radicaux hydrocarbonés en C₇-C₂₁, linéaires ou ramifiés, saturés ou insaturés ;
      - n, p et r, identiques ou différents, sont des entiers valant de 2 à 6 ;
      - y est un entier valant de 1 à 10 ;
      - x et z, identiques ou différents, sont des entiers valant de 0 à 10 ;
      - X⁻ est un anion simple ou complexe, organique ou inorganique ;
      sous réserve que la somme x + y + z vaut de 1 à 15 , que lorsque x vaut 0 alors R₁₆ désigne R₂₀ et que lorsque z vaut 0 alors R₁₈ désigne R₂₂.

On utilise plus particulièrement les sels d'ammonium de formule (XVI) dans laquelle :
- R₁₅ désigne un radical méthyle ou éthyle,
- x et y sont égaux à 1 ;
- z est égal à 0 ou 1 ;
- n, p et r sont égaux à 2 ;
- R₁₆ est choisi parmi :
   - le radical 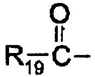
   - les radicaux méthyle, éthyle ou hydrocarbonés en C₁₄-C₂₂
   - l'atome d'hydrogène ;
- R₁₇, R₁₉ et R₂₁, identiques ou différents, sont choisis parmi les radicaux hydrocarbonés en C₇-C₂₁, linéaires ou ramifiés, saturés ou insaturés ;
- R₁₈ est choisi parmi :
   - le radical 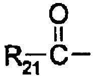
   - l'atome d'hydrogène ;

De tels composés sont par exemple commercialisés sous les dénominations DEHYQUART par la société COGNIS, STEPANQUAT par la société STEPAN, NOXAMIUM par la société CECA, REWOQUAT WE 18 par la société REWO-WITCO.

Parmi les sels d'ammonium quaternaire on préfère le chlorure de béhényltriméthylammonium, ou encore, le chlorure de stéaramidopropyldiméthyl (myristyl acétate) ammonium commercialisé sous la dénomination «CERAPHYL 70» par la société VAN DYK, le Quaternium-27 ou le Quaternium-83 commercialisés par la société WITCO.

Dans les compositions conformes à l'invention, on peut utiliser des mélanges d'agents tensioactifs et en particulier des mélanges d'agents tensioactifs anioniques, des mélanges d'agents tensioactifs anioniques et d'agents tensioactifs amphotères, cationiques ou non ioniques, des mélanges d'agents tensioactifs cationiques avec des agents tensioactifs non ioniques ou amphotères. Un mélange particulièrement préféré est un mélange comprenant au moins un agent tensioactif anionique et au moins un agent tensioactif amphotère.

Le ou les agents tensioactifs sont généralement présents à une concentration de 0,01% à 50% en poids, de préférence de 0,1% à 40%, plus préférentiellement de 0,5% à 30% en poids, par rapport au poids total de la composition.

La composition de l'invention peut également contenir au moins un additif choisi parmi les épaississants, les parfums, les agents nacrants, les conservateurs, les filtres solaires siliconés ou non, les polymères anioniques ou non ioniques, les protéines non cationiques ou pas, les hydrolysats de protéines non cationiques ou pas, l'acide méthyl-18 eicosanoique, les hydroxyacides, les vitamines, les provitamines teilles que le panthénol, les silicones, les agents antipelliculaires ou anti-séborrhéiques, les électrolytes, les protéines, les hydrolysats de protéines, les huiles fluorées ou perfluorées, les silicones, les cires naturelles ou synthétiques, les composés de type céramide, les amines grasses, les acides gras et leurs dérivés, les alcools gras et leurs dérivés ainsi que les mélanges de ces différents composés et tout autre additif classiquement utilisé dans le domaine cosmétique qui n'affecte pas les propriétés des compositions selon l'invention.

Les compositions conformes à l'invention peuvent également contenir jusqu'à 5 % d'agents nacrants ou opacifiants bien connus dans l'état de la technique tels que par exemple les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les éthers à chaînes grasses notamment en C10-C30 tels que par exemple le distéaryléther ou le 1-(hexadécyloxy)-2-octadécanol, les alcools gras en particulier les alcools stéarylique, cétylique, béhénylique et leurs mélanges.

Ces additifs sont éventuellement présents dans la composition selon l'invention dans des proportions pouvant aller de 0,001 à 20% en poids par rapport au poids total de la composition. La quantité précise de chaque additif est déterminée facilement par l'homme du métier selon sa nature et sa fonction.

Le milieu physiologiquement et notamment cosmétiquement acceptable peut être constitué uniquement par de l'eau ou par un mélange d'eau et d'un solvant cosmétiquement acceptable tel qu'un alcool inférieur en C₁-C₄, comme l'éthanol, l'isopropanol, le tertiobutanol, le n-butanol ; les alkylèneglycols comme le propylèneglycol, les éthers de glycols.

De préférence, la composition comprend de 50 à 95 % en poids d'eau par rapport au poids total de la composition, et plus particulièrement de 60 à 90% en poids.

Les compositions selon l'invention présentent un pH final généralement compris entre 3 et 10. De préférence, ce pH est compris entre 4 et 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide minéral ou organique, de préférence un acide carboxylique tel que par exemple l'acide citrique.

Les compositions conformes à l'invention peuvent être plus particulièrement utilisées pour le lavage ou le traitement des matières kératiniques telles que les cheveux, la peau, les cils, les sourcils, les ongles, les lèvres, le cuir chevelu et plus particulièrement les cheveux.

Les compositions selon l'invention peuvent être des compositions détergentes telles que des shampooings, des gels-douche et des bains moussants. Dans ce mode de réalisation de l'invention, les compositions comprennent au moins une base lavante, généralement aqueuse.

Le ou les tensioactifs constituant la base lavante peuvent être indifféremment choisis, seuls ou en mélanges, au sein des tensioactifs anioniques, amphotères, non ioniques et cationiques tels que définis ci-dessus. La base lavante contient au moins un tensioactif détergent.

Dans les compositions conformes à l'invention, on utilise de préférence au moins un ou plusieurs tensioactifs anioniques ou des mélanges d'au moins un ou plusieurs tensioactifs anioniques et d'au moins un ou plusieurs tensioactifs amphotères ou d'au moins un ou plusieurs tensioactifs non ioniques.

Un mélange particulièrement préféré est un mélange comprenant au moins un agent tensioactif anionique et au moins un agent tensioactif amphotère.

On utilise de préférence un agent tensioactif anionique choisi parmi les alkyl(C₁₂-C₁₄) sulfates de sodium, de triéthanolamine ou d'ammonium, les alkyl (C₁₂-C₁₄)éthersulfates de sodium, de triéthanolamine ou d'ammonium oxyéthylénés à 2,2 moles d'oxyde d'éthylène, le cocoyl iséthionate de sodium et l'alphaoléfine(C₁₄-C₁₆) sulfonate de sodium et leurs mélange avec :
- soit un agent tensioactif amphotère tel que les dérivés d'amine dénommés disodiumcocoamphodipropionate ou sodiumcocoamphopropionate commercialisés notamment par la société RHODIA CHIMIE sous la dénomination commerciale "MIRANOL C2M CONC" en solution aqueuse à 38 % de matière active ou sous la dénomination MIRANOL C32;
- soit un agent tensioactif amphotère de type zwittérionique tel que les alkylbétaïnes ou les alkylamidobétaïnes et en particulier la cocobétaïne commercialisée sous la dénomination "DEHYTON AB 30" en solution aqueuse à 32 % de MA par la société COGNIS.

On utilise de préférence un agent tensioactif anionique choisi parmi les alkyl(C₁₂-C₁₄) sulfates de sodium, de triéthanolamine ou d'ammonium, les alkyl (C₁₂-C₁₄)éthersulfates de sodium, de triéthanolamine ou d'ammonium oxyéthylénés à 2,2 moles d'oxyde d'éthylène, le cocoyl iséthionate de sodium et l'alphaoléfine(C₁₄-C₁₆) sulfonate de sodium et leurs mélange avec :
- soit un agent tensioactif amphotère tel que les dérivés d'amine dénommés disodiumcocoamphodipropionate ou sodiumcocoamphopropionate commercialisés notamment par la société RHODIA CHIMIE sous la dénomination commerciale "MIRANOL C2M CONC" en solution aqueuse à 38 % de matière active ou sous la dénomination MIRANOL C32;
- soit un agent tensioactif amphotère de type zwittérionique tel que les alkylbétaïnes en particulier la cocobétaïne commercialisée sous la dénomination "DEHYTON AB 30" en solution aqueuse à 32 % de MA par la société COGNIS et la cocoamidopropylbétaïne vendue notamment par GOLDSCHMIDT sous le nom de TEGOBETAINE F50.

La quantité et la qualité de la base lavante sont celles suffisantes pour conférer à la composition finale un pouvoir moussant et/ou détergent satisfaisant.

Ces compositions détergentes sont de préférence moussante et le pouvoir moussant des compositions selon l'invention, caractérisé par une hauteur de mousse, est généralement supérieur à 75 mm ; de préférence, supérieure à 100 mm mesurée selon la méthode ROSS-MILES (NF T 73-404 /ISO696) modifiée.

Les modifications de la méthode sont les suivantes :
La mesure se fait à la température de 22°C avec de l'eau osmosée. La concentration de la solution est de 2g/l. La hauteur de la chute est de 1m. La quantité de composition qui chute est de 200 ml. Ces 200 ml de composition tombe dans une éprouvette ayant un diamètre de 50 mm et contenant 50 ml de la composition à tester. La mesure est faite 5 minutes après l'arrêt de l'écoulement de la composition.

Ainsi, selon l'invention, la base lavante peut représenter de 3 % à 50 % en poids, de préférence de 6 % à 35 % en poids, et encore plus préférentiellement de 8 % à 25 % en poids, du poids total de la composition finale.

L'invention a encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage à l'eau.

Ainsi, ce procédé selon l'invention permet le maintien de la coiffure, le traitement, le soin ou le lavage ou le démaquillage de la peau, des cheveux ou de toute autre matière kératinique.

Les compositions de l'invention peuvent également se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Lorsque la composition se présente sous la forme d'un après-shampooing éventuellement à rincer, elle contient avantageusement au moins un tensioactif cationique, sa concentration généralement comprise entre 0,1 et 10% en poids et de préférence de 0,5 à 5% en poids par rapport au poids total de la composition.

Les compositions de l'invention peuvent encore se présenter sous la forme de compositions lavantes pour la peau, et en particulier sous la forme de solutions ou de gels pour le bain ou la douche ou de produits démaquillants.

Les compositions selon l'invention peuvent également se présenter sous forme de lotions aqueuses ou hydroalcooliques pour le soin de la peau et/ou des cheveux.

Les compositions cosmétiques selon l'invention peuvent se présenter sous forme de gel, de lait, de crème, d'émulsion, de lotion épaissie ou de mousse et être utilisées pour la peau, les ongles, les cils, les lèvres et plus particulièrement les cheveux.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des matières kératiniques en particulier les cheveux.

Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits. Dans les exemples, MA signifie matière active.

### EXEMPLE 1

On a réalisé un shampooing conforme à l'invention de composition suivante :

| Composition | Exemple |
|---|---|
| Lauryléthersulfate de sodium à 2.2 moles d'oxyde d'éthylène en solution aqueuse à 70% de MA | 12.5 g M.A. |
| Cocoyl bétaïne en solution aqueuse à 30% de MA | 2.4 g M.A. |
| Alkyl (C₈/C₁₀/C₁₂/C₁₄ 34/24/29/10) polyglucoside (1,4) | 1.4 g M.A. |
| Copolymère réticulé acide méthacrylique / acrylate d'éthyle en émulsion aqueuse à 30% de matière active commercialisé par NOVEON sous la dénomination Carbopol AQUA SF-1 | 1.2 g M.A. |
| Huile d'avocat | 1 g |
| Gomme de guar modifiée par du chlorure de 2,3-époxypropyl triméthyl ammonium, vendue sous la dénomination Jaguar C13S par la société RHODIA CHIMIE | 0.1 g |
| Distéarate d'éthylèneglycol | 2 g |
| Alcool cétylstéarylique oxyéthyléné (60 OE) éther de myristyl glycol | 1.5 g |
| Conservateurs | q.s. |
| Acide citrique ou soude q.s. | pH 5.5 |
| Eau déminéralisée q.s.p. | 100 g |

La composition est stable. Les cheveux mouillés ne sont pas chargés et la mise en forme est aisée.

## Revendications

1. Composition cosmétique, **caractérisée par le fait qu'**elle comprend, dans un milieu cosmétiquement acceptable, au moins un copolymère réticulé d'acide méthacrylique et d'acrylate d'alkyle en C₁-C₄, au moins une huile choisie parmi les huiles synthétiques choisies parmi les polyoléfines, les esters d'acides en C₃-C₁₄ et comprenant au total moins de 20 atomes de carbone, les huiles végétales ayant une teneur en acide palmitoléique supérieure ou égale à 0,2% en poids de l'huile, et un polymère cationique.

2. Composition selon la revendication 1, **caractérisée en ce que** dans ledit copolymère, l'acide méthacrylique est présent dans des quantités allant de 20 à 80% en poids et plus particulièrement de 25 à 70 % en poids et encore plus particulièrement de 35 à 60% en poids par rapport au poids total du copolymère.

3. Composition selon la revendication 1 ou 2, **caractérisée en ce que** dans ledit copolymère, l'acrylate d'alkyle est présent dans des quantités allant de 15 à 80% en poids et plus particulièrement de 25 à 75 % en poids et encore plus particulièrement de 40 à 65% en poids par rapport au poids total du copolymère.

4. Composition selon l'une quelconque des revendications 1 à 3, **caractérisée en ce que** dans ledit copolymère, l'acrylate d'alkyle est choisi parmi l'acrylate de méthyle, l'acrylate d'éthyle ou l'acrylate de butyle.

5. Composition selon la revendication 4, **caractérisée en ce que** l'acrylate d'alkyle est l'acrylate d'éthyle.

6. Composition selon l'une quelconque des revendications 1 à 5, où le copolymère d'acide méthacrylique et d'acrylate d'alkyle en C₁-C₄ est réticulé par au moins un agent réticulant éthylèniquement polyinsaturé.

7. Composition selon la revendication 6, **caractérisée en ce que** la teneur en agent réticulant varie de 0,01 à 5% en poids et de préférence de 0,03 à 3% en poids et encore plus particulièrement de 0,05 à 1% en poids par rapport au poids total du copolymère.

8. Composition selon l'une quelconque des revendications 1 à 7, **caractérisée en ce que** le copolymère d'acide méthacrylique et d'acrylate d'alkyle en C₁-C₄ est sous forme de dispersion de particules dans l'eau.

9. Composition selon la revendication 8, **caractérisée en ce que** la taille moyenne en nombre des particules de copolymère dans la dispersion varie de 10 à 500 nm et de préférence de 20 à 200 nm et plus préférentiellement de 50 à 150 nm.

10. Composition selon l'une quelconques des revendications 1 à 9, **caractérisée par le fait que** les huiles de synthèse sont choisies parmi les poly-α-oléfines.

11. Composition selon la revendication 10, **caractérisée par le fait que** les poly-α-oléfines sont:
- de type polybutène, hydrogéné ou non, et de préférence polyisobutène, hydrogéné ou non.
- de type polydécène, hydrogéné ou non.

12. Composition selon l'une quelconques des revendications 1 à 9, **caractérisée par le fait que** les huiles végétales sont choisies dans le groupe formé par les huiles d'avocat, de jojoba, d'olive, d'abricot et les huiles végétales transestérifiées.

13. Composition selon l'une quelconques des revendications 1 à 9, **caractérisée par le fait que** lesdits esters sont choisis parmi les esters d'acides mono- di-, tri-carboxyliques aliphatiques saturés ou insaturés, linéaires ou ramifiés en C₃-C₁₄ et de monoalcools ou de polyols aliphatiques, saturés ou insaturés, linéaires ou ramifiés en C₁-C₁₆, le nombre total de carbone étant inférieur ou égal à 20.

14. Composition selon la revendication 13, **caractérisée par le fait que** lesdits esters sont choisis parmi le lactate de cétyle ; le lactate d'alkyle en C₁₂-C₁₅; le lactate de lauryle ; le lactate d'oléyle ; les myristates d'alkyles en C₁-C₅ tels que le myristate d'isopropyle, de butyle, le laurate d'hexyle, l'isononate d'isononyle, le néopentanoate d'isodécyle.

15. Composition selon l'une quelconque des revendications précédentes, **caractérisée par le fait que** les polymères cationiques sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

16. Composition selon l'une quelconque des revendications précédentes, **caractérisée par le fait que** ledit polymère cationique est choisi parmi :
(1) les homopolymères ou copolymères dérivés d'esters ou d'amides acryliques ou méthacryliques et comportant au moins un des motifs de formules suivantes: 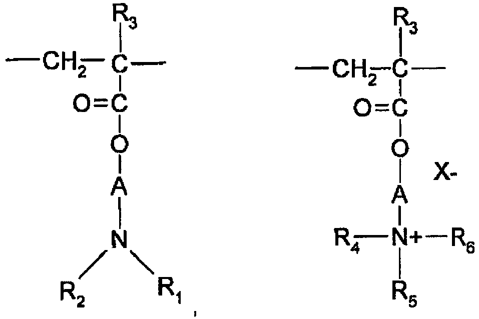 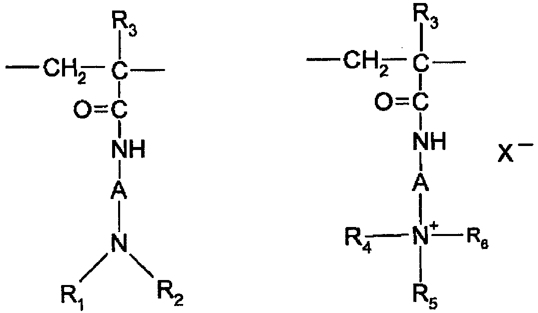 dans lesquelles:
R₃, identiques ou différents, désignent un atome d'hydrogène ou un radical CH₃;
A, identiques ou différents, représentent un groupe alkyle, linéaire ou ramifié, de 1 à 6 atomes de carbone ou un groupe hydroxyalkyle de 1 à 4 atomes de carbone ;
R₄, R₅, R₆, identiques ou différents, représentent un groupe alkyle ayant de 1 à 18 atomes de carbone ou un radical benzyle;
R₁ et R₂, identiques ou différents, représentent hydrogène ou un groupe alkyle ayant de 1 à 6 atomes de carbone;
X désigne un anion dérivé d'un acide minéral ou organique.
(2) Les polysaccharides cationiques,
(3) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quatemisation de ces polymères,
(4) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées.
(5) les dérivés de polyaminoamides résultant de la condensation de polyalcoylènes polyamines avec des acides polycarboxyliques suivie d'une alcoylation par des agents bifonctionnels,
(6) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone,
(7) les cyclopolymères d'alkyl diallyl amine ou de dialkyl diallyl ammonium
(8) les polymères de diammonium quaternaire contenant des motifs récurrents répondant à la formule : 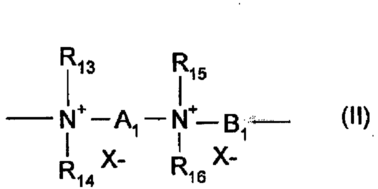 formule (II) dans laquelle :
R13, R14, R15 et R16, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R13, R14, R15 et R16, ensemble ou séparément, constituent avec les atomes d'azote auxquels ils sont rattachés des hétérocycles contenant éventuellement un second hétéroatome autre que l'azote ou bien R13, R14, R15 et R16 représentent un radical alkyle en C1-C6 linéaire ou ramifié substitué par un groupement nitrile, ester, acyle, amide ou -CO-O-R17-D ou -CO-NH-R17-D où R17 est un alkylène et D un groupement ammonium quaternaire ;
A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
X⁻ désigne un anion dérivé d'un acide minéral ou organique;
A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
dans lequel D désigne :
a) un reste de glycol de formule : -O-Z-O-, où Z désigne un radical hydrocarboné linéaire ou ramifié ou un groupement répondant à l'une des formules suivantes :
-(CH2-CH2-O)x-CH2-CH2-
-[CH2-CH(CH3)-O]y-CH2-CH(CH3)-
où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine :
c) un reste de diamine bis-primaire de formule : -NH-Y-NH-, où Y désigne un radical hydrocarboné linéaire ou ramifié, ou bien le radical bivalent
-CH2-CH2-S-S-CH2-CH2- ;
d) un groupement uréylène de formule : -NH-CO-NH-;
De préférence, X⁻ est un anion.
(9) les polymères de polyammonium quaternaires comprenant des motifs de formule (III): 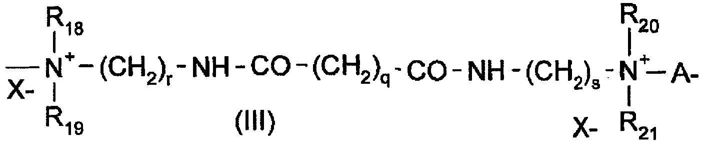 formule dans laquelle :
R18, R19, R20 et R21, identiques ou différents, représentent un atome d'hydrogène ou un radical méthyle, éthyle, propyle, β-hydroxyéthyle, β-hydroxypropyle ou
-CH2CH2(OCH2CH2)pOH,
où p est égal à 0 ou à un nombre entier compris entre 1 et 6, sous réserve que R18, R19, R20 et R21 ne représentent pas simultanément un atome d'hydrogène,
r et s, identiques ou différents, sont des nombres entiers compris entre 1 et 6,
q est égal à 0 ou à un nombre entier compris entre 1 et 34,
X désigne un atome d'halogène,
A désigne un radical d'un dihalogénure ou représente de préférence -CH2-CH2-O-CH2-CH2-.
(10) Les polymères quaternaires de vinylpyrrolidone et de vinylimidazole,
(11) Les polyamines référencées sous le nom de « POLYETHYLENEGLYCOL (15) TALLOW POLYAMINE » dans le dictionnaire CTFA.
(12) Les polymères réticulés de sels de méthacryloyloxyalkyl(C₁-C₄) trialkyl(C₁-C₄)ammonium,
(13) des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

17. Composition selon la revendication précédente, **caractérisée par le fait que** ledit polymère cationique est choisi parmi les cyclopolymères cationiques, les polysaccharides cationiques, les polymères quaternaires de vinylpyrrolidone et de vinylimidazole, les homopolymères ou copolymères réticulés de sels de méthacryloyloxyalkyl(C₁-C₄) trialkyl(C₁-C₄)ammonium et leurs mélanges.

18. Composition selon la revendication 17, **caractérisée par le fait que** ledit cyclopolymère est choisi parmi les homopolymères du chlorure de diallyldiméthylammonium et les copolymères du chlorure de diallyldiméthylammonium et d'acrylamide.

19. Composition selon la revendication 17, **caractérisée par le fait que** lesdits polysaccharides cationiques sont choisis parmi les gommes de guar modifiées par un sel de 2,3-époxypropyl triméthylammonium et les hydroxyéthylcelluloses ayant réagi avec un époxyde substitué par un groupement triméthylammonium.

20. Composition selon l'une quelconque des revendications précédentes, **caractérisée en ce que** le copolymère réticulé acide méthacrylique et d'acrylate d'alkyle en C₁-C₄ est présent à une concentration comprise entre 0,01 % et 20 % en poids par rapport au poids total de la composition, de préférence entre 0,05 % et 15 % en poids.

21. Composition selon l'une quelconque des revendications précédentes, **caractérisée en ce que** l'huile est présente à une concentration comprise entre 0,001 % et 20 % en poids par rapport au poids total de la composition, de préférence entre 0,01 % et 10 % en poids.

22. Composition selon l'une quelconque des revendications 15 à 21, **caractérisée en ce que** le polymère cationique est présent à une concentration comprise entre 0,001 % et 20 % en poids par rapport au poids total de la composition, de préférence entre 0,01 % et 10 % en poids.

23. Composition selon l'une quelconque des revendications précédentes, **caractérisée par le fait qu'**elle comprend en outre au moins un agent tensioactif choisi parmi les tensioactifs anioniques, non ioniques, amphotères, cationiques et leurs mélanges.

24. Composition selon la revendication 23, **caractérisée par le fait que** le ou les agents tensioactifs sont choisis parmi au moins un ou plusieurs tensioactifs anioniques ou des mélanges d'au moins un ou plusieurs tensioactifs anioniques et d'au moins un ou plusieurs tensioactifs amphotères ou d'au moins un ou plusieurs tensioactifs non ioniques.

25. Composition selon l'une quelconque des revendications 23 ou 24, **caractérisée par le fait que** le ou les agents tensioactifs sont présents à une concentration comprise entre 0,01% et 50% en poids, de préférence entre 0,1% et 40% en poids, et encore plus préférentiellement entre 0,5% et 30% en poids, par rapport au poids total de la composition.

26. Composition selon l'une quelconque des revendications 1 à 25, **caractérisée par le fait que** la composition contient au moins un additif choisi parmi les épaississants, les agents antipelliculaires ou anti-séborrhéiques, les parfums, les agents nacrants, les hydroxyacides, les électrolytes, les conservateurs, les filtres solaires siliconés ou non, les vitamines, les provitamines telles que le panthénol, les polymères anioniques ou non ioniques, les protéines, les hydrolysats de protéines, l'acide méthyl-18 eicosanoique, les huiles fluorées ou perfluorées, les silicones, les cires naturelles ou synthétiques, les composés de type céramide, les amines grasses, les acides gras et leurs dérivés, les alcools gras et leurs dérivés ainsi que les mélanges de ces différents composés.

27. Composition selon l'une quelconque des revendications précédentes, **caractérisée par le fait qu'**elle se présente sous forme de shampooing, d'après-shampooing, de composition pour la permanente, le défrisage, la coloration ou la décoloration des cheveux, de composition à rincer à appliquer entre les deux étapes d'une permanente ou d'un défrisage, de composition lavantes pour le corps.

28. Utilisation d'une composition telle que définie dans l'une quelconque des revendications précédentes pour le lavage ou pour le soin des matières kératiniques.

29. Procédé de traitement des matières kératiniques, telles que les cheveux, **caractérisé en ce qu'**il consiste à appliquer sur lesdites matières une composition cosmétique selon l'une des revendications 1 à 27, puis à effectuer éventuellement un rinçage à l'eau.

30. Utilisation d'un copolymère réticulé acide méthacrylique/acrylate en C₁-C₄, dans, ou pour la fabrication d'une composition cosmétique comprenant une huile telle que définie à l'une des revendications 1 et 10 à 14, et un polymère cationique.

31. Utilisation d'une composition telle que définie dans l'une quelconque des revendications 1 à 27 pour apporter de la légèreté aux cheveux.

32. Utilisation d'une composition telle que définie dans l'une quelconque des revendications 1 à 27 pour apporter de la douceur aux cheveux.

33. Utilisation d'une composition telle que définie dans l'une quelconque des revendications 1 à 27 pour apporter, du lissage au toucher aux cheveux.

34. Utilisation d'une composition telle que définie dans l'une quelconque des revendications 1 à 27 pour apporter de la souplesse aux cheveux.

## Patentansprüche

1. Kosmetische Zusammensetzung, **dadurch gekennzeichnet, dass** sie in einem kosmetisch akzeptablen Medium mindestens ein vernetztes Copolymer von Methacrylsäure und C₁₋₄-Alkylacrylat, mindestens ein Öl, ausgewählt aus den synthetischen Ölen, ausgewählt aus den Polyolefinen, den Estern der C₃-C₁₄-Fettsäuren mit insgesamt weniger als 20 Kohlenstoffatomen und den Pflanzenölen mit einem Palmitoleinsäuregehalt von 0,2 Gew.-% des Öls oder mehr sowie ein kationisches Polymer umfasst.

2. Zusammensetzung nach Anspruch 1, **dadurch gekennzeichnet, dass** die Methacrylsäure in dem Copolymer in Mengenanteilen von 20 bis 80 Gew.-%, insbesondere 25 bis 70 Gew.-% und besonders 35 bis 60 Gew.-%, bezogen auf das Gesamtgewicht des Copolymers, enthalten ist.

3. Zusammensetzung nach Anspruch 1 oder 2, **dadurch gekennzeichnet, dass** das Alkylacrylat in dem Copolymer in Mengenanteilen von 15 bis 80 Gew.-%, insbesondere 25 bis 75 Gew.-% und besonders 40 bis 65 Gew.-%, bezogen auf das Gesamtgewicht des Copolymers, enthalten ist.

4. Zusammensetzung nach einem der Ansprüche 1 bis 3, **dadurch gekennzeichnet, dass** in dem Copolymer das Alkylacrylat unter Methylacrylat, Ethylacrylat oder Butylacrylat ausgewählt ist.

5. Zusammensetzung nach Anspruch 4, **dadurch gekennzeichnet, dass** das Alkylacrylat das Ethylacrylat ist.

6. Zusammensetzung nach einem der Ansprüche 1 bis 5, wobei das Copolymer von Methacrylsäure und C₁₋₄-Alkylacrylat mit mindestens einem mehrfach ethylenisch ungesättigten Vernetzungsmittel vernetzt ist.

7. Zusammensetzung nach Anspruch 6, **dadurch gekennzeichnet, dass** der Gehalt des Vernetzungsmittels im Bereich von 0,01 bis 5 Gew.-%, vorzugsweise 0,03 bis 3 Gew.-% und noch bevorzugter 0,05 bis 1 Gew.-%, bezogen auf das Gesamtgewicht des Copolymers, liegt.

8. Zusammensetzung nach einem der Ansprüche 1 bis 7, **dadurch gekennzeichnet, dass** das Copolymer von Methacrylsäure und C₁₋₄-Alkylacrylat in Form einer Dispersion von Partikeln in Wasser vorliegt.

9. Zusammensetzung nach Anspruch 8, **dadurch gekennzeichnet, dass** die zahlenmittlere Größe der Copolymerpartikel in der Dispersion im Bereich von 10 bis 500 nm, vorzugsweise 20 bis 200 nm und noch bevorzugter 50 bis 150 nm liegt.

10. Zusammensetzung nach einem der Ansprüche 1 bis 9, **dadurch gekennzeichnet, dass** die synthetischen Öle unter den Poly-α-olefinen ausgewählt sind.

11. Zusammensetzung nach Anspruch 10, **dadurch gekennzeichnet, dass** die Poly-α-olefine:
- vom hydrierten oder nicht hydrierten Polybutylentyp, vorzugsweise vom hydrierten oder nicht hydrierten Polyisobutentyp;
- vom hydrierten oder nicht hydrierten Polydecentyp sind.

12. Zusammensetzung nach einem der Ansprüche 1 bis 9, **dadurch gekennzeichnet, dass** die pflanzlichen Öle unter der Gruppe bestehend aus Avocadoöl, Jojobaöl, Olivenöl, Aprikosenkernöl und umgeesterten pflanzlichen Ölen ausgewählt sind.

13. Zusammensetzung nach einem der Ansprüche 1 bis 9, **dadurch gekennzeichnet, dass** die Ester unter den Estern von gesättigten oder ungesättigten, geradkettigen oder verzweigten aliphatischen Mono-, Di- und Tricarbonsäuren mit 3 bis 14 Kohlenstoffatomen und gesättigten oder ungesättigten, geradkettigen oder verzweigten aliphatischen Monoalkoholen oder Polyolen mit 1 bis 16 Kohlenstoffatomen ausgewählt sind, wobei die Gesamtzahl der Kohlenstoffatome höchstens 20 beträgt.

14. Zusammensetzung nach Anspruch 13, **dadurch gekennzeichnet, dass** die Ester unter Cetyllactat; C₁₂₋₁₅-Alkyllactat; Lauryllactat; Oleyllactat; C₁₋₅-Alkylmyristaten, wie Isopropylmyristat, Butylmyristat, Hexyllaurat, Isononylisononat, Isodecylneopentanoat ausgewählt sind.

15. Zusammensetzung nach einem der vorhergehenden Ansprüche, **dadurch gekennzeichnet, dass** die kationischen Polymere unter den Polymeren ausgewählt sind, die Einheiten enthalten, die primäre, sekundäre, tertiäre und/oder quartäre Aminogruppen aufweisen, die entweder Teil der Polymerhauptkette sein können oder von einem direkt daran gebundenen seitlichen Substituenten getragen werden.

16. Zusammensetzung nach einem der vorhergehenden Ansprüche, **dadurch gekennzeichnet, dass** das kationische Polymer ausgewählt ist unter:
(1) Homopolymeren oder Copolymeren, die von Acrylsäure- oder Methacrylsäureestern oder Acryl- oder Methacrylamiden abgeleitet sind und mindestens eine der Einheiten der folgenden Formeln enthalten: 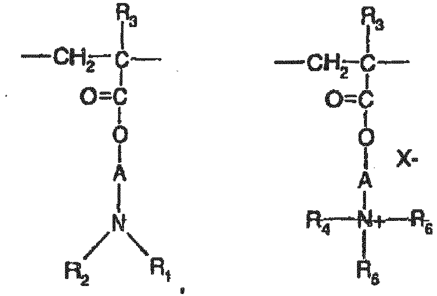 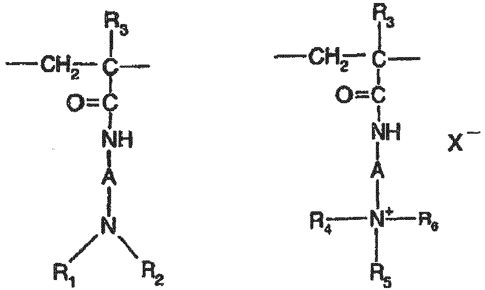 worin:
die Gruppen R₃, die gleich oder verschieden sind, ein Wasserstoffatom oder die Gruppe CH₃ bedeuten; die Gruppen A, die gleich oder verschieden sind, eine geradkettige oder verzweigte Alkylgruppe mit 1 bis 6 Kohlenstoffatomen oder eine Hydroxyalkylgruppe mit 1 bis 4 Kohlenstoffatomen bedeuten;
die Gruppen R₄, R₅ und R₆, die gleich oder verschieden sind, eine Alkylgruppe mit 1 bis 18 Kohlenstoffatomen oder eine Benzylgruppe bedeuten; die Gruppen R₁ und R₂, die gleich oder verschieden sind, Wasserstoff oder eine Alkylgruppe mit 1 bis 6 Kohlenstoffatomen bedeuten;
X ein von einer anorganischen oder organischen Säure abgeleitetes Anion bezeichnet;
(2) kationischen Polysacchariden;
(3) Polymeren, die aus Piperazinyleinheiten und zweiwertigen Alkylen- oder Hydroxyalkylengruppen mit geraden oder verzweigten Ketten gebildet sind, die gegebenenfalls durch Sauerstoffatome, Schwefelatome, Stickstoffatome oder aromatische oder heteroaromatische Ringe unterbrochen sind, sowie den Oxidationsprodukten und/oder Quarternisierungsprodukten dieser Polymere;
(4) in Wasser löslichen Polyaminoamiden, die insbesondere durch Polykondensation einer sauren Verbindung mit einem Polyamin hergestellt sind; diese Polyaminoamide können mit einem Epihalohydrin, Diepoxid, Dianhydrid, ungesättigten Dianhydrid, zweifach ungesättigten Derivat, Bis-halohydrin, Bis-azetidinium, Bis-haloacyldiamin, Alkyl-bis-halogenid oder auch einem Oligomer vernetzt sein, das bei der Umsetzung einer bifunktionellen Verbindung entsteht, die gegenüber einem Bis-halohydrin, Bis-azetidinium, Bis-haloacyldiamin, Alkyl-bis-halogenid, Epihalohydrin, Diepoxid oder zweifach ungesättigten Derivat reaktiv ist; wobei das Vernetzungsmittel in Mengenanteilen von 0,025 bis 0,35 Mol pro Aminogruppe des Polyaminoamids verwendet wird; wobei diese Polyaminoamide alkyliert sein können oder, wenn sie eine oder mehrere tertiäre Aminofunktionen aufweisen, quaternisiert sein können,
(5) Polyaminoamidderivaten, die bei der Kondensation von Polyalkylenpolyaminen mit Polycarbonsäuren und anschließender Alkylierung mit bifunktionellen Mitteln gebildet werden,
(6) Polymeren, die durch Umsetzung eines Polyalkylenpolyamins mit zwei primären Aminogruppen und mindestens einer sekundären Aminogruppe mit einer Dicarbonsäure gebildet werden, die unter Diglycolsäure und den aliphatischen gesättigten Dicarbonsäuren mit 3 bis 8 Kohlenstoffatomen ausgewählt ist,
(7) Alkyldiallylamincyclopolymeren oder Dialkyldiallylammoniumcyclopolymeren,
(8) quartären Diammoniumpolymeren, die Wiederholungseinheiten der folgenden Formel enthalten: 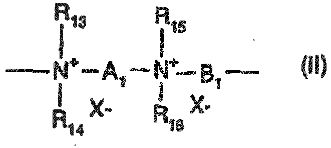 wobei in der Formel (II):
die Gruppen R13, R14, R15 und R16, die gleich oder verschieden sind, aliphatische, alicyclische oder arylaliphatische Gruppen mit 1 bis 20 Kohlenstoffatomen oder niedere hydroxyalkylaliphatische Gruppen bedeuten oder die Gruppen R13, R14, R15 und R16 gemeinsam oder getrennt voneinander mit den Stickstoffatomen, an die sie gebunden sind, Heterocyclen bilden, die gegebenenfalls ein zweites, von Stickstoff verschiedenes Heteroatom enthalten, oder die Gruppen R13, R14, R15 und R16 eine geradkettige oder verzweigte C₁₋₆-Alkylgruppe bedeuten, die mit einer Nitrilgruppe, Estergruppe, Acylgruppe, Amidgruppe oder -CO-O-R17-D oder -CO-NH-R17-D substituiert ist, wobei R17 eine Alkylengruppe ist und D eine quartäre Ammoniumgruppe bedeutet;
die Gruppen A1 und B1 Polymethylengruppen mit 2 bis 20 Kohlenstoffatomen bedeuten, die geradkettig oder verzweigt, gesättigt oder ungesättigt sein können und in der Hauptkette oder an die Hauptkette gebunden einen oder mehrere aromatische Ringe oder ein oder mehrere Sauerstoffatome, Schwefelatome oder Sulfoxidgruppen, Sulfongruppen, Disulfidgruppen, Aminogruppen, Alkylaminogruppen, Hydroxygruppen, quartäre Ammoniumgruppen, Ureidogruppen, Amidgruppen oder Estergruppen enthalten können; und
X⁻ ein Anion ist, das von einer anorganischen oder organischen Säure abgeleitet ist;
wobei die Gruppen A1, R13 und R15 mit den beiden Stickstoffatomen, an die sie gebunden sind, einen Piperazinring bilden können; wenn A1 eine geradkettige oder verzweigte, gesättigte oder ungesättigte Alkylen- oder Hydroxyalkylengruppe ist, kann B1 ferner eine Gruppe (CH2)n-CO-D-OC-(CH2)n- bedeuten,
worin D:
a) einen Glycolrest der Formel: -O-Z-O-, wobei Z eine geradkettige oder verzweigte Kohlenwasserstoffgruppe oder eine Gruppe einer der folgenden Formeln bedeutet:
-(CH2-CH2-O)x-CH2-CH2-
-[CH2-CH(CH3)-O]y-CH2-CH(CH3)-
wobei x und y eine ganze Zahl von 1 bis 4 bedeuten und einen definierten und einzigen Polymerisationsgrad angeben oder eine beliebige Zahl von 1 bis 4 und einen mittleren Polymerisationsgrad bedeuten;
b) einen bis-sekundären Diaminrest, beispielsweise ein Piperazinderivat;
c) einen bis-primären Diaminrest der Formel: -NH-Y-NH-, wobei Y eine geradkettige oder verzweigte Kohlenwasserstoffgruppe oder die zweiwertige Gruppe
-CH2-CH2-S-S-CH2-CH2- bedeutet;
d) eine Ureylengruppe der Formel: -NH-CO-NH-;
wobei X⁻ vorzugsweise ein Anion ist; bedeutet.
(9) quartären Polyammoniumpolymeren, die Einheiten der Formel (III) enthalten: 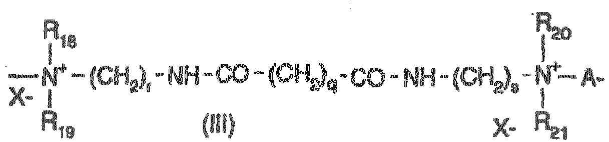 wobei in der Formel:
die Gruppen R18, R19, R20 und R21, die gleich oder verschieden sind, ein Wasserstoffatom oder Methyl, Ethyl, Propyl, β-Hydroxyethyl, β-Hydroxypropyl oder -CH2CH2(OCH2CH2)ₚOH bedeuten,
wobei p 0 oder eine ganze Zahl von 1 bis 6 ist, mit der Maßgabe, dass die Gruppen R18, R19, R20 und R21 nicht gleichzeitig Wasserstoff bedeuten,
r und s, die gleich oder verschieden sind, ganze Zahlen von 1 bis 6 sind,
q 0 oder eine ganze Zahl von 1 bis 34 ist,
X ein Halogenatom bedeutet,
A eine Dihalogenidgruppe oder vorzugsweise
-CH2-CH2-O-CH2-CH2- bedeutet,
(10) quartären Polymeren von Vinylpyrrolidon und Vinylimidazol,
(11) Polyaminen, die nach CTFA-Nomenklatur als "POLYETHYLENGLYCOL (15) TALLOW POLYAMINE" bezeichnet werden,
(12) vernetzten Polymeren von Methacryloyloxyalkyl (C₁₋₄) trialkyl- (C₁₋₄) ammoniumsalzen,
(13) Polyalkyleniminen, insbesondere Polyethyleniminen, Polymeren, die Vinylpyridin- oder Vinylpyridiniumeinheiten enthalten, Kondensaten von Polyaminen und Epichlorhydrin, quartären Polyureylenen und Chitinderivaten.

17. Zusammensetzung nach dem vorhergehenden Anspruch, **dadurch gekennzeichnet, dass** das kationische Polymer unter den kationischen Cyclopolymeren, kationischen Polysacchariden, quartären Polymeren von Vinylpyrrolidon und Vinylimidazol, vernetzten Homopolymeren oder Copolymeren von Methacryloyloxyalkyl (C₁₋₄) trialkyl (C₁₋₄) ammoniumsalzen und deren Gemischen ausgewählt ist.

18. Zusammensetzung nach Anspruch 17, **dadurch gekennzeichnet, dass** das Cyclopolymer unter den Homopolymeren von Diallyldimethylammoniumchlorid und den Copolymeren von Diallyldimethylammoniumchlorid und Acrylamid ausgewählt ist.

19. Zusammensetzung nach Anspruch 17, **dadurch gekennzeichnet, dass** die kationischen Polysaccharide unter den mit einem 2,3-Epoxypropyltrimethylammoniumsalz modifizierten Guargummen und Hydroxyethylcellulosen ausgewählt sind, die mit einem mit einer Trimethylammoniumgruppe substituierten Epoxid umgesetzt wurden.

20. Zusammensetzung nach einem der vorhergehenden Ansprüche, **dadurch gekennzeichnet, dass** das vernetzte Copolymer von Methacrylsäure und C₁₋₄-Alkylacrylat in einer Konzentration von 0,01 bis 20 Gew.-%, bezogen auf das Gesamtgewicht der Zusammensetzung, und vorzugsweise 0,05 bis 15 Gew.-%, enthalten ist.

21. Zusammensetzung nach einem der vorhergehenden Ansprüche, **dadurch gekennzeichnet, dass** das Öl in einer Konzentration von 0,001 bis 20 Gew.-%, bezogen auf das Gesamtgewicht der Zusammensetzung, und vorzugsweise 0,01 bis 10 Gew.-% enthalten ist.

22. Zusammensetzung nach einem der Ansprüche 15 bis 21, **dadurch gekennzeichnet, dass** das kationische Polymer in einer Konzentration von 0,001 bis 20 Gew.-%, bezogen auf das Gesamtgewicht der Zusammensetzung, und vorzugsweise 0,01 bis 10 Gew.-% enthalten ist.

23. Zusammensetzung nach einem der vorhergehenden Ansprüche, **dadurch gekennzeichnet, dass** sie ferner mindestens einen grenzflächenaktiven Stoff enthält, der unter den anionischen, nichtionischen, amphoteren, kationischen grenzflächenaktiven Stoffen und deren Gemischen ausgewählt ist.

24. Zusammensetzung nach Anspruch 23, **dadurch gekennzeichnet, dass** der oder die grenzflächenaktiven Stoffe unter mindestens einem oder mehreren anionischen grenzflächenaktiven Stoffen oder Gemischen aus mindestens einem oder mehreren anionischen grenzflächenaktiven Stoffen und mindestens einem oder mehreren amphoteren grenzflächenaktiven Stoffen oder mindestens einem oder mehreren nichtionischen grenzflächenaktiven Stoffen ausgewählt sind.

25. Zusammensetzung nach einem der Ansprüche 23 oder 24, **dadurch gekennzeichnet, dass** der oder die grenzflächenaktiven Stoffe in einer Konzentration von 0,01 bis 50 Gew.-%, vorzugsweise 0,1 bis 40 Gew.-% und noch bevorzugter 0,5 bis 30 Gew.-%, bezogen auf das Gesamtgewicht der Zusammensetzung, enthalten sind.

26. Zusammensetzung nach einem der Ansprüche 1 bis 25, **dadurch gekennzeichnet, dass** die Zusammensetzung mindestens einen Zusatzstoff enthält, der unter den Verdickungsmitteln, Antischuppenmitteln oder Mitteln gegen Seborrhoe, Parfums, Perlglanzstoffen, Hydroxysäuren, Elektrolyten, Konservierungsmitteln, siliconierten oder nicht siliconierten Sonnenschutzfiltern, Vitaminen, Provitaminen wie Panthenol, anionischen oder nichtionischen Polymeren, Proteinen, Proteinhydrolysaten, 18-Methyleicosansäure, fluorierten oder perfluorierten Ölen, Siliconen, natürlichen oder synthetischen Wachsen, Verbindungen vom Ceramidtyp, Fettaminen, Fettsäuren und deren Derivaten, Fettalkoholen und deren Derivaten sowie den Gemischen dieser verschiedenen Verbindungen ausgewählt ist.

27. Zusammensetzung nach einem der vorhergehenden Ansprüche, **dadurch gekennzeichnet, dass** sie als Haarwaschmittel, Produkt, das nach der Haarwäsche angewandt wird, Zusammensetzung für permanente Verformungen, Entkräuselungen, Färbungen oder Entfärbungen der Haare, Zusammensetzung, die ausgespült wird und zwischen den beiden Schritten einer dauerhaften Verformung oder Entkräuselung aufgebracht wird, reinigende Zusammensetzung für den Körper vorliegt.

28. Verwendung einer Zusammensetzung nach einem der vorhergehenden Ansprüche für die Reinigung oder die Pflege von Keratinsubstanzen.

29. Verfahren zur Behandlung von Keratinsubstanzen, wie Haaren, **dadurch gekennzeichnet, dass** es darin besteht, auf die Substanzen eine kosmetische Zusammensetzung nach einem der Ansprüche 1 bis 27 aufzutragen und anschließend gegebenenfalls mit Wasser zu spülen.

30. Verwendung eines vernetzten Methacrylsäure/C₁₋₄-Acrylatpolymers in einer kosmetischen Zusammensetzung, die ein Öl wie es in einem der Ansprüche 1 und 10 bis 14 definiert ist und ein kationisches Polymer enthält, oder für die Herstellung einer solchen Zusammensetzung.

31. Verwendung einer Zusammensetzung nach einem der Ansprüche 1 bis 27, um den Haaren Lockerheit zu geben.

32. Verwendung einer Zusammensetzung nach einem der Ansprüche 1 bis 27, um den Haaren Weichheit zu geben.

33. Verwendung einer Zusammensetzung nach einem der Ansprüche 1 bis 27, damit sich die Haare glatt anfühlen.

34. Verwendung einer Zusammensetzung nach einem der Ansprüche 1 bis 27, um den Haaren Geschmeidigkeit zu geben.

## Claims

1. Cosmetic composition, **characterized in that** it comprises, in a cosmetically acceptable medium, at least one crosslinked copolymer of methacrylic acid and of a C₁-C₄ alkyl acrylate, at least one oil chosen from synthetic oils chosen from polyolefins, C₃-C₁₄ acid esters comprising in total less than 20 carbon atoms, and plant oils with a palmitoleic acid content of greater than or equal to 0.2% by weight of the oil, and a cationic polymer.

2. Composition according to Claim 1, **characterized in that**, in the said copolymer, the methacrylic acid is present in amounts ranging from 20% to 80% by weight and more particularly from 25% to 70% by weight and even more particularly from 35% to 60% by weight relative to the total weight of the copolymer.

3. Composition according to Claim 1 or 2, **characterized in that**, in the said copolymer, the alkyl acrylate is present in amounts ranging from 15% to 80% by weight and more particularly from 25% to 75% by weight and even more particularly from 40% to 65% by weight relative to the total weight of the copolymer.

4. Composition according to any one of Claims 1 to 3, **characterized in that**, in the said copolymer, the alkyl acrylate is chosen from methyl acrylate, ethyl acrylate or butyl acrylate.

5. Composition according to Claim 4, **characterized in that** the alkyl acrylate is ethyl acrylate.

6. Composition according to any one of Claims 1 to 5, in which the copolymer of methacrylic acid and of a C₁-C₄ alkyl acrylate is crosslinked with at least one ethylenically polyunsaturated crosslinking agent.

7. Composition according to Claim 6, **characterized in that** the crosslinking agent content ranges from 0.01% to 5% by weight and preferably from 0.03% to 3% by weight and even more particularly from 0.05% to 1% by weight relative to the total weight of the copolymer.

8. Composition according to any one of Claims 1 to 7, **characterized in that** the copolymer of methacrylic acid and of a C₁-C₄ alkyl acrylate is in the form of a dispersion of particles in water.

9. Composition according to Claim 8, **characterized in that** the number-average size of the copolymer particles in the dispersion ranges from 10 to 500 nm and preferably from 20 to 200 nm and more preferably from 50 to 150 nm.

10. Composition according to any one of Claims 1 to 9, **characterized in that** the synthetic oils are chosen from poly-α-olefins.

11. Composition according to Claim 10, **characterized in that** the poly-α-olefins are:
- of hydrogenated or non-hydrogenated polybutene type, and preferably hydrogenated or non-hydrogenated polyisobutene,
- of hydrogenated or non-hydrogenated polydecene type.

12. Composition according to any one of Claims 1 to 9, **characterized in that** the plant oils are chosen from the group formed by avocado oil, jojoba oil, olive oil, apricot oil and transesterified plant oils.

13. Composition according to any one of Claims 1 to 9, **characterized in that** the said esters are chosen from esters of saturated or unsaturated, linear or branched C₃-C₁₄ aliphatic mono-, di- and tricarboxylic acids and of saturated or unsaturated, linear or branched C₁-C₁₆ aliphatic monoalcohols or polyols, the total carbon number being less than or equal to 20.

14. Composition according to Claim 13, **characterized in that** the said esters are chosen from cetyl lactate; C₁₂-C₁₅ alkyl lactate; lauryl lactate; oleyl lactate; C₁-C₅ alkyl myristates such as isopropyl myristate or butyl myristate, hexyl laurate, isononyl isononate and isodecyl neopentanoate.

15. Composition according to any one of the preceding claims, **characterized in that** the cationic polymers are chosen from those containing units comprising primary, secondary, tertiary and/or quaternary amine groups that may either form part of the main polymer chain, or be borne by a side substituent directly attached to the said chain.

16. Composition according to any one of the preceding claims, **characterized in that** the said cationic polymer is chosen from:
(1) homopolymers or copolymers derived from acrylic or methacrylic esters or amides and comprising at least one of the units of the following formulae: 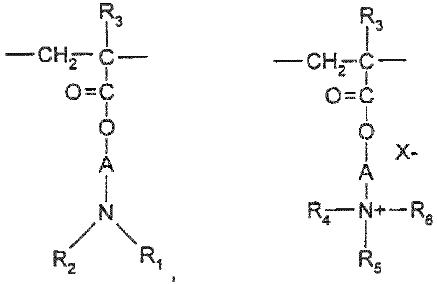 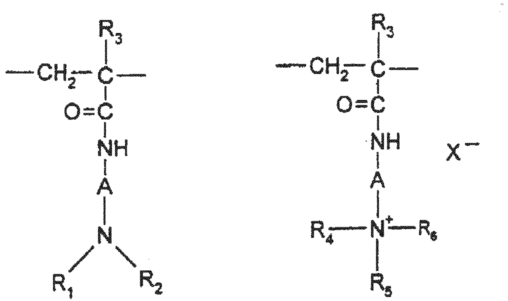 in which:
R₃, which may be identical or different, denote a hydrogen atom or a CH₃ radical;
A, which may be identical or different, represent a linear or branched alkyl group of 1 to 6 carbon atoms, or a hydroxyalkyl group of 1 to 4 carbon atoms;
R₄, R₅ and R₆, which may be identical or different, represent an alkyl group containing from 1 to 18 carbon atoms or a benzyl radical;
R₁ and R₂, which may be identical or different, represent hydrogen or an alkyl group containing from 1 to 6 carbon atoms;
X denotes an anion derived from an inorganic or organic acid.
(2) cationic polysaccharides,
(3) polymers consisting of piperazinyl units and of divalent alkylene or hydroxyalkylene radicals containing straight or branched chains, optionally interrupted by oxygen, sulphur or nitrogen atoms or by aromatic or heterocyclic rings, as well as the oxidation and/or quaternization products of these polymers,
(4) water-soluble polyamino amides prepared in particular by polycondensation of an acidic compound with a polyamine; these polyamino amides can be crosslinked with an epihalohydrin, a diepoxide, a dianhydride, an unsaturated dianhydride, a bis-unsaturated derivative, a bis-halohydrin, a bis-azetidinium, a bis-haloacyldiamine, a bis-alkyl halide or alternatively with an oligomer resulting from the reaction of a difunctional compound which is reactive with a bis-halohydrin, a bis-azetidinium, a bis-haloacyldiamine, a bis-alkyl halide, an epihalohydrin, a diepoxide or a bis-unsaturated derivative; the crosslinking agent being used in proportions ranging from 0.025 to 0.35 mol per amine group of the polyamino amide; these polyamino amides can be alkylated or, if they contain one or more tertiary amine functions, they can be quaternized.
(5) polyamino amide derivatives resulting from the condensation of polyalkylene polyamines with polycarboxylic acids followed by an alkylation with difunctional agents,
(6) polymers obtained by reaction of a polyalkylene polyamine containing two primary amine groups and at least one secondary amine group with a dicarboxylic acid chosen from diglycolic acid and saturated aliphatic dicarboxylic acids having from 3 to 8 carbon atoms,
(7) cyclopolymers of alkyldiallylamine or of dialkyldiallylammonium,
(8) quaternary diammonium polymers containing repeating units corresponding to the formula: 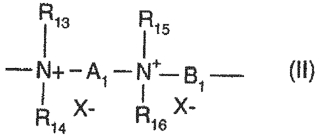 in which formula (II):
R₁₃, R₁₄, R₁₅ and R₁₆, which may be identical or different, represent aliphatic, alicyclic or arylaliphatic radicals containing from 1 to 20 carbon atoms or lower hydroxyalkylaliphatic radicals, or alternatively R₁₃, R₁₄, R₁₅ and R₁₆, together or separately, constitute, with the nitrogen atoms to which they are attached, heterocycles optionally containing a second hetero atom other than nitrogen, or alternatively R₁₃, R₁₄, R₁₅ and R₁₆ represent a linear or branched C₁-C₆ alkyl radical substituted with a nitrile, ester, acyl or amide group or a group -CO-O-R₁₇-D or -CO-NH-R₁₇-D where R₁₇ is an alkylene and D is a quaternary ammonium group;
A₁ and B₁ represent polymethylene groups containing from 2 to 20 carbon atoms, which groups may be linear or branched, saturated or unsaturated, and which may contain, linked to or intercalated in the main chain, one or more aromatic rings or one or more oxygen or sulphur atoms or sulphoxide, sulphone, disulphide, amino, alkylamino, hydroxyl, quaternary ammonium, ureido, amide or ester groups, and
X- denotes an anion derived from an inorganic or organic acid;
A₁, R₁₃ and R₁₅ can form, with the two nitrogen atoms to which they are attached, a piperazine ring; in addition, if A₁ denotes a linear or branched, saturated or unsaturated alkylene or hydroxyalkylene radical, B₁ can also denote a group (CH₂)ₙ-CO-D-OC-(CH₂)ₙ-
in which D denotes:
a) a glycol residue of formula: -O-Z-O-, where Z denotes a linear or branched hydrocarbon-based radical or a group corresponding to one of the following formulae:
- (CH₂-CH₂-O)ₓ-CH₂-CH₂-
- [CH₂-CH(CH₃)-O]_{y}-CH₂-CH(CH₃) -
where x and y denote an integer from 1 to 4, representing a defined and unique degree of polymerization or any number from 1 to 4 representing an average degree of polymerization;
b) a bis-secondary diamine residue such as a piperazine derivative;
c) a bis-primary diamine residue of formula: -NH-Y-NH-, where Y denotes a linear or branched hydrocarbon radical, or alternatively the divalent radical
-CH₂-CH₂-S-S-CH₂-CH₂- ;
d) a ureylene group of formula: -NH-CO-NH-;
preferably, X⁻ is an anion,
(9) polyquaternary ammonium polymers comprising units of formula (III): 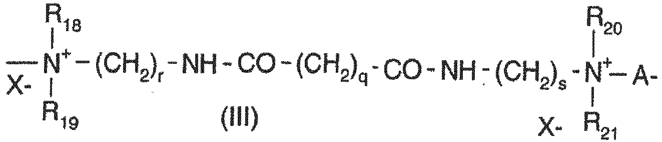 in which formula:
R₁₈, R₁₉, R₂₀ and R₂₁, which may be identical or different, represent a hydrogen atom or a methyl, ethyl, propyl, β-hydroxyethyl, β-hydroxypropyl or -CH₂CH₂ (OCH₂CH₂)ₚOH radical,
where p is equal to 0 or to an integer between 1 and 6, with the proviso that R₁₈, R₁₉, R₂₀ and R₂₁ do not simultaneously represent a hydrogen atom,
r and s, which may be identical or different, are integers between 1 and 6,
q is equal to 0 or to an integer between 1 and 34,
X denotes a halogen atom,
A denotes a dihalide radical or preferably represents -CH₂-CH₂-O-CH₂-CH₂-,
(10) quaternary polymers of vinylpyrrolidone and of vinylimidazole,
(11) the polyamines referenced under the name "Polyethylene glycol (15) tallow polyamine" in the CTFA dictionary,
(12) crosslinked polymers of methacryloyloxy-(C₁-C₄) alkyltri (C₁-C₄) alkylammonium salts,
(13) polyalkyleneimines, in particular polyethyleneimines, polymers containing vinylpyridine or vinylpyridinium units, condensates of polyamines and of epichlorohydrin, quaternary polyureylenes and chitin derivatives.

17. Composition according to the preceding claim, **characterized in that** the said cationic polymer is chosen from cationic cyclopolymers, cationic polysaccharides, quaternary polymers of vinylpyrrolidone and of vinylimidazole, crosslinked homopolymers or copolymers of methacryloyloxy (C₁-C₄) alkyltri (C₁-C₄) alkylammonium salts, and mixtures thereof.

18. Composition according to Claim 17, **characterized in that** the said cyclopolymer is chosen from diallyldimethylammonium chloride homopolymers and copolymers of diallyldimethylammonium chloride and of acrylamide.

19. Composition according to Claim 17, **characterized in that** the said cationic polysaccharides are chosen from guar gums modified with a 2,3-epoxypropyltrimethylammonium salt and hydroxyethylcelluloses that have reacted with an epoxide substituted with a trimethylammonium group.

20. Composition according to any one of the preceding claims, **characterized in that** the crosslinked copolymer of methacrylic acid and of a C₁-C₄ alkyl acrylate is present in a concentration of between 0.01% and 20% by weight relative to the total weight of the composition and preferably between 0.05% and 15% by weight.

21. Composition according to any one of the preceding claims, **characterized in that** the oil is present in a concentration of between 0.001% and 20% by weight, relative to the total weight of the composition and preferably between 0.01% and 10% by weight.

22. Composition according to any one of Claims 15 to 21, **characterized in that** the cationic polymer is present in a concentration of between 0.001% and 20% by weight relative to the total weight of the composition and preferably between 0.01% and 10% by weight.

23. Composition according to any one of the preceding claims, **characterized in that** it also comprises at least one surfactant chosen from anionic, nonionic, amphoteric and cationic surfactants, and mixtures thereof.

24. Composition according to Claim 23, **characterized in that** the surfactant(s) is (are) chosen from at least one or more anionic surfactants or mixtures of at least one or more anionic surfactants and of at least one or more amphoteric surfactants or of at least one or more nonionic surfactants.

25. Composition according to either of Claims 23 and 24, **characterized in that** the surfactant(s) is (are) present in a concentration of between 0.01% and 50% by weight, preferably between 0.1% and 40% by weight and even more preferably between 0.5% and 30% by weight relative to the total weight of the composition.

26. Composition according to any one of Claims 1 to 25, **characterized in that** the composition contains at least one additive chosen from thickeners, antidandruff agents, anti-seborrhoeic agents, fragrances, nacreous agents, hydroxy acids, electrolytes, preserving agents, silicone or non-silicone sunscreens, vitamins, provitamins such as panthenol, anionic or nonionic polymers, proteins, protein hydrolysates, 18-methyleicosanoic acid, fluoro or perfluoro oils, silicones, natural or synthetic waxes, compounds of ceramide type, fatty amines, fatty acids and derivatives thereof, fatty alcohols and derivatives thereof and also mixtures of these various compounds.

27. Composition according to any one of the preceding claims, **characterized in that** it is in the form of a shampoo, a conditioner, a permanent-waving, straightening, dyeing or bleaching composition for the hair, a rinse-out composition to be applied between the two steps of a permanent-waving or hair-straightening operation, or a washing composition for the body.

28. Use of a composition as defined in any one of the preceding claims, for washing or caring for keratin materials.

29. Process for treating keratin materials, such as the hair, **characterized in that** it consists in applying to the said materials a cosmetic composition according to one of Claims 1 to 27, and then optionally rinsing it out with water.

30. Use of a crosslinked methacrylic acid/C₁-C₄ acrylate copolymer in, or for the manufacture of, a cosmetic composition comprising an oil as defined in one of Claims 1 and 10 to 14, and a cationic polymer.

31. Use of a composition as defined in any one of Claims 1 to 27, to give the hair lightness.

32. Use of a composition as defined in any one of Claims 1 to 27, to give the hair softness.

33. Use of a composition as defined in any one of Claims 1 to 27, to give the hair a smooth feel.

34. Use of a composition as defined in any one of Claims 1 to 27, to give the hair flexibility.
